(19) Europäisches Patentamt

European Patent Office

Office européen des brevets

(11) Veröffentlichungsnummer: **0 290 768 B1**

(12) **EUROPÄISCHE PATENTSCHRIFT**

(45) Veröffentlichungstag der Patentschrift: **19.01.94**

(21) Anmeldenummer: **88104952.2**

(22) Anmeldetag: **28.03.88**

(51) Int. Cl.⁵: **C12N 15/53**, C12N 9/04, C12N 1/21, C12Q 1/32, //(C12N1/20,C12R1:19)

(54) Verfahren zur Herstellung von Glucosedehydrogenase aus Bacillus megaterium.

(30) Priorität: **08.04.87 DE 3711881**

(43) Veröffentlichungstag der Anmeldung:
**17.11.88 Patentblatt 88/46**

(45) Bekanntmachung des Hinweises auf die Patenterteilung:
**19.01.94 Patentblatt 94/03**

(84) Benannte Vertragsstaaten:
**CH DE ES FR GB IT LI NL SE**

(56) Entgegenhaltungen:
**EP-A- 0 095 950**
**EP-A- 0 285 949**

**FEBS LETT., Band 165, Nr. 1, January 1984, Seiten 6-10, Federation of EuropeanBioche-mical Societies; K.-D. JANY et al.: "Complete amino acid sequence ofglucose dehydroge-nase from Bacillus megaterium"**

**PROC. NATL. ACAD. SCI. USA, Band 80, February 1983, Seiten 785-789; N. VASANTHA**

(73) Patentinhaber: **MERCK PATENT GmbH**
**Frankfurter Strasse 250**
**D-64293 Darmstadt(DE)**

(72) Erfinder: **Ebeling, Wolfgang, Dr.**
**Am Hintergraben 9**
**D-6101 Bickenbach(DE)**
Erfinder: **Heilmann, Hans-Jürgen, Dr.**
**Schöneweibergasse 91**
**D-6103 Griesheim(DE)**
Erfinder: **Meinhardt, Friedhelm, Dr.**
**Gross-Erckenschwickerstrasse 95**
**D-4353 Oer-Erckenschick(DE)**

EP 0 290 768 B1

et al.: "Isolation of a developmental gene of Bacillus subtilis and itsexpression in Escherichia coli"

J. THEOR. BIOL., Band 120, Nr. 4, 1986, Seiten 489-497, Academic Press Inc.(London) Ltd; P. FORTNAGEL et al.: "Sequence homologies of glucose-dehydrogenases of Bacillus megaterium and Bacillus subtilis"

BIOLOGICAL ABSTRACTS, Nr. 80021199; E. DE ROCCA-SERRA et al.: "Glucose assay inhemolysate and biological fluids by a micromethod using glucose dehydrogenase",& FEUILLETS DE BIOLOGIE(FRANCE) 1984,vol. 25, no. 140, p49-56

CHEMICAL ABSTRACTS, Band 84, 1976, Seite 160, Zusammenfassung Nr. 1639m,Columbus, Ohio, US; H.E. PAULY et al.: "D-glucose dehydrogenase from Bacillusmegaterium M 1286. Purification, properties, and structure",& HOPPE-SEYLER'S Z. PHYSIOL. CHEM. 1975, 356(10), 1613-23

CHEMICAL ABSTRACTS, Band 83, 4. August 1975, Seite 226, Zusammenfassung Nr.39778y, Columbus, Ohio, US; D. BANAUCH et al.: "Glucose dehydrogenase for thedetermination of glucose concentrations in body fluids",

& Z. KLIN. CHEM. KLIN. BIOCHEM. 1975, 13(3), 101-7

EUR. J. BIOCHEM., Band 174, Nr. 3, 1988, Seiten 485-490, FEBS; H.J. HEILMANN etal.: "Identification and isolation of glucose dehydrogenase genes of Bacillusmegaterium M1286 and their expression in Escherichia coli"

**Beschreibung**

Die Erfindung betrifft ein gentechnisches Verfahren zur Herstellung von Glucose-Dehydrogenase. Dabei wird aus einem kultivierten mikrobiellen Wirtsorganismus das Enzym Glucose-Dehydrogenase isoliert, nachdem der Wirt zuvor mit entsprechender rekombinanter DNA transformiert wurde. Die rekombinante DNA enthält eine Sequenz, die aus einem Genom vom Bacillus megaterium stammt und für ein Polypeptid mit der biologischen Aktivität der Glucose-Dehydrogenase codiert.

Die Erfindung betrifft weiterhin die Bereitstellung einer DNA-Sequenz, die für ein Polypeptid mit der biologischen Aktivität des Enzyms Glucose-Dehydrogenase codiert.

Außerdem betrifft die Erfindung Klonierungs- und Expressionsvektoren zur Verwendung bei der Herstellung eines Polypeptids mit der biologischen Aktivität des Enzyms Glucose-Dehydrogenase sowie mit solchen Vektoren transformierte Wirtsorganismen, beispielsweise Bakterien, Hefen, andere Pilze, tierische oder menschliche Zellen.

Schließlich betrifft die Erfindung die Verwendung der zur Expression gebrachten entsprechenden Polypeptide für die Bestimmung von Glucose. Der Ausdruck "Polypeptid mit der biologischen Aktivität des Enzyms Glucose-Dehydrogenase" bezeichnet ein Polypeptid bzw. Protein, dessen Aminosäure-Sequenz der nativen Glucose-Dehydrogenase aus Bacillus megaterium-Stämmen entspricht, ähnlich zu dieser Sequenz ist oder einen enzymatisch aktiven Teilbereich umfaßt. Analoges gilt auch für solche erfindungsgemäßen Polypeptide, in denen die native Sequenz nur einen Teilbereich darstellt.

NAD/NADP-abhängige Glucose-Dehydrogenase (E.C. 1.1.1.47) im folgenden Glucose-DH benannt, katalysiert die Umsetzung von $\beta$-Glucose zu Gluconolacton, wobei der Co-Faktor NAD zu $NADH_2$ reduziert wird. Das Enzym zeichnet sich durch hohe Substratspezifität in Bezug auf $\beta$-D-Glucose aus. Epimere Zucker werden nicht umgesetzt. Die Hauptanwendung des Enzyms liegt in der medizinischen Diagnostik. NAD-abhängige Glucose-DH kommt in einer Reihe von Bacillusarten vor. Die Bildung dieses Enzyms unterliegt einer entwicklungsphysiologischen Steuerung, d.h. es wird nur während einer kurzen Phase der Sporulation synthetisiert. Zudem ist Glucose-DH in den meisten Fällen mit $NADH_2$-Oxidasen vergesellschaftet, welche die Bestimmung von Glucose nach der o.g. Glucose-DH-Methode empfindlich stören und sich zudem nur schwer entfernen lassen. Dies alles hat zur Folge, daß de Ausbeuten an Enzym mittels üblicher klassischer Methoden und unter Verwendung von Bacillus-Wildstämmen sehr bescheiden sind (0,01 bis max. 0,1 Units/ml Kultur). Mit Hilfe bekannter Stammverbesserungs-Methoden lassen sich Stämme selektieren, bei denen eine Glucose-DH-Bildung auch unabhängig von der Sporulationsphase feststellbar ist (durchschnittliche Enzymaktivitäten 1 bis 10 Units/ml Kultur), jedoch können solche "entkoppelten" Stämme leicht revertieren, d.h. zum ursprünglichen Sporulationsverhalten zurückkehren, so daß ein steter und oftmals langwieriger Aufwand betrieben werden muß, um Sporen- und Glucose-DH-Bildung voneinander getrennt zu halten. Zwar gelang es kürzlich (Vasantha et al., 1983, Proc. Natl. Acad. Science, USA, Vol. 80, 785-789) ein Glucose-DH codierendes Gen einschließlich seines Promotors aus Bacillus subtilis zu isolieren und in E. coli sporulationsunabhängig zur Expression zu bringen, jedoch erwies sich dieses Enzym im Gegensatz zu der im Handel erhältlichen Glucose-DH überraschenderweise als recht instabil (Ramaley, Vasantha 1983, J. Biol. Chem., Vol. 258, 20, 12558-12565). Hohe Enzymstabilität ist aber gerade erforderlich, um einen schnellen und reproduzierbaren Glucose-Bestimmungstest durchführen zu können.

Der Erfindung liegt somit die Aufgabe zugrunde, ein gentechnologisches Verfahren bereitzustellen, das die technische Herstellung einer stabilen Glucose-Dehydrogenase in großen Mengen und in verbesserter Qualität ermöglicht. Diese Aufgabe wurde wie nachstehend angegeben gelöst.

Überraschend wurde gefunden, daß durch Anlegen einer Genbank von Bacillus megaterium in einem Phagenvektor, vorzugsweise λ-EMBL-3, sukzessive Verkleinerung des das Strukturgen tragenden DNA-Bereichs und jeweilige Transformation eines Wirtsorganismus, insbesondere E. coli, mindestens zwei unterschiedliche Strukturgene getrennt erhalten werden können, die letztlich zur Expression von mindestens zwei unterschiedlichen Glucose-DH-Isoenzymen in verbesserter Qualität und Quantität führen.

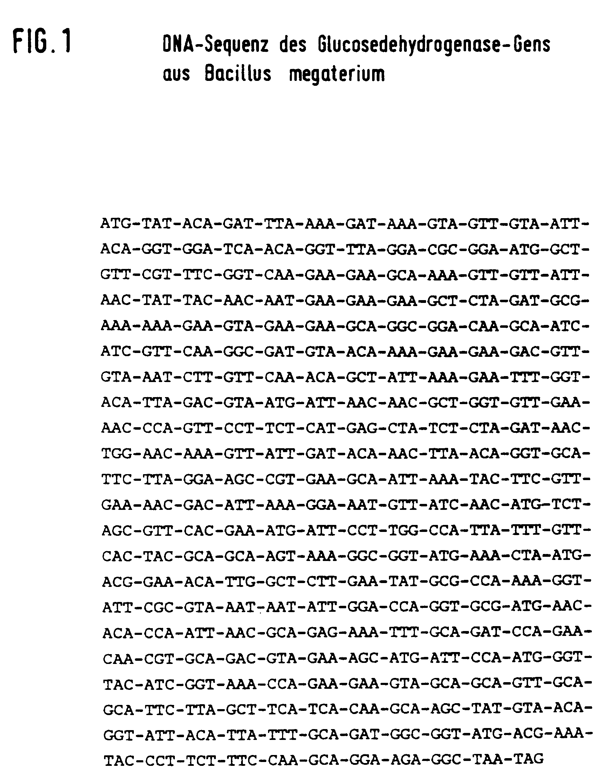

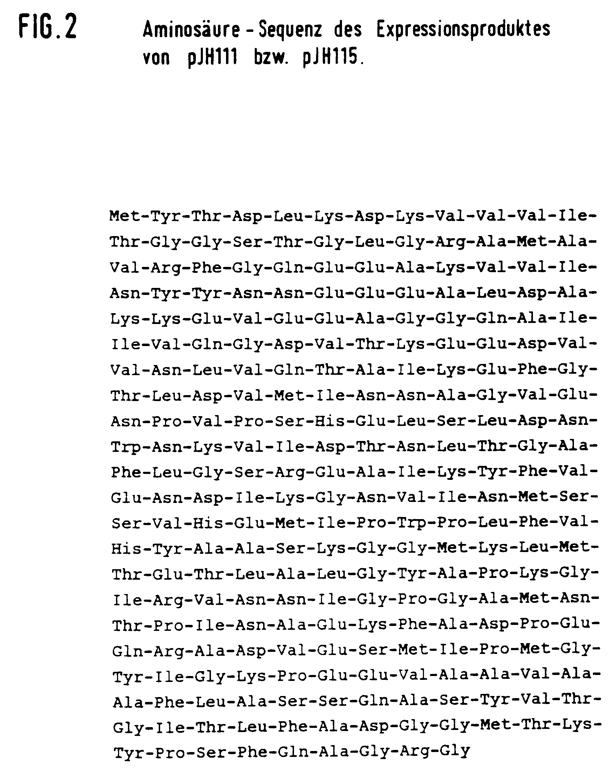

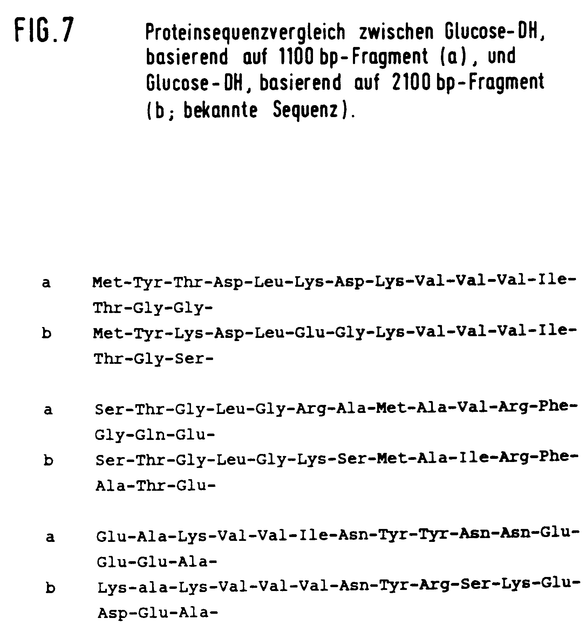

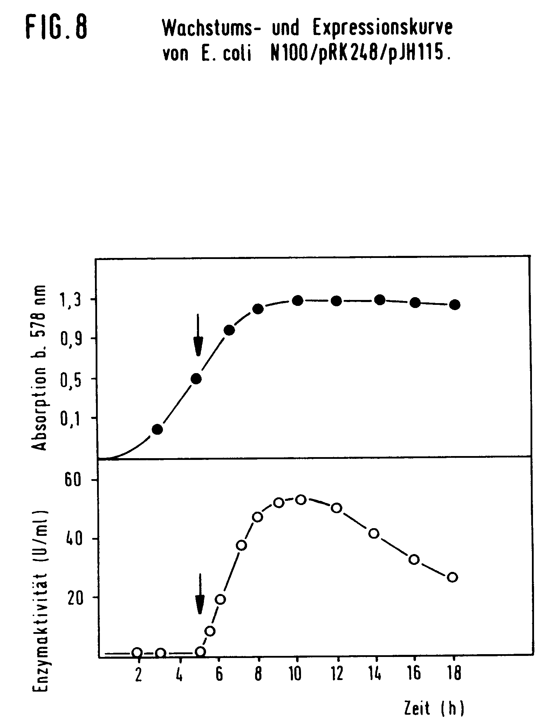

Während der eine identifizierte Glucose-DH codierende, 2100 Basenpaare (bp) große DNA-Bereich in DNA- und Aminosäuresequenz der bekannten Sequenz von käuflicher Glucose-DH voll entspricht (Jany et al., 1984, FEBS Lett. 165; 6-10), enthält das andere 1100 bp große DNA-Fragment die in Figur 1 dargestellte neue Sequenz. Die davon abgeleitete und durch Proteinsequenzierung des aufgereinigten Enzyms bestätigte neue Aminosäuresequenz ist in Figur 2 wiedergegeben. Die Abweichung gegenüber der bekannten Sequenz beträgt fast 20 %. Die Unterschiede in den ersten 45 Aminosäuren sind in Figur 7 dargestellt. Baut man diese verschiedenen DNA-Fragmente in hierfür geeignete Plasmidvektoren (z. B. pJH111 und pJH211) ein und transformiert entsprechende Wirtsorganismen, vorzugsweise E. coli-Zellen, so erhält man allein durch einen in E. coli aktiven bacilluseigenen Promotor in beiden Fällen Glucose-DH-Enzymaktivitäten von 0.05 bis 0.5 Units/ml Kultur, vorzugsweise 0.09 bis 0.2 Units/ml Kultur. Dies übertrifft die Werte, die nach klassischen Methoden für Bacillus-Wildstämme erreicht werden können, bereits mind. um den Faktor 5. Durch Einbau des bekannten regulierbaren $\lambda$-$P_L$-Promotors vor das jeweilige Strukturgen lassen sich überraschend hohe Enzymaktivitäten messen: 30-65 Units/ml Kultur, vorzugsweise 40-50 Units/ml Kultur bei Verwendung des neuen Polypeptids gemäß Figur 2 bei E. coli N100/pRK248/pJH115, und 20-40 Units/ml Kultur, vorzugsweise 25-35 Units/ml Kultur bei Verwendung des Polypeptids der bekannten Sequenz bei E. coli N100/pRK248/pJH215. Diese Werte liegen ca. 500mal höher als die Glucose-DH-Ausbeute bei Bacillus-Wildstämmen und ca. 5-10mal höher als bei den aufwendig zu entwik-kelnden sporulationsentkoppelten Bacillus-Stämmen. Eine typische Wachstums- und Expressionskurve ist in Figur 8 exemplarisch für E. coli N100/pRK248/pJH115 wiedergegeben.

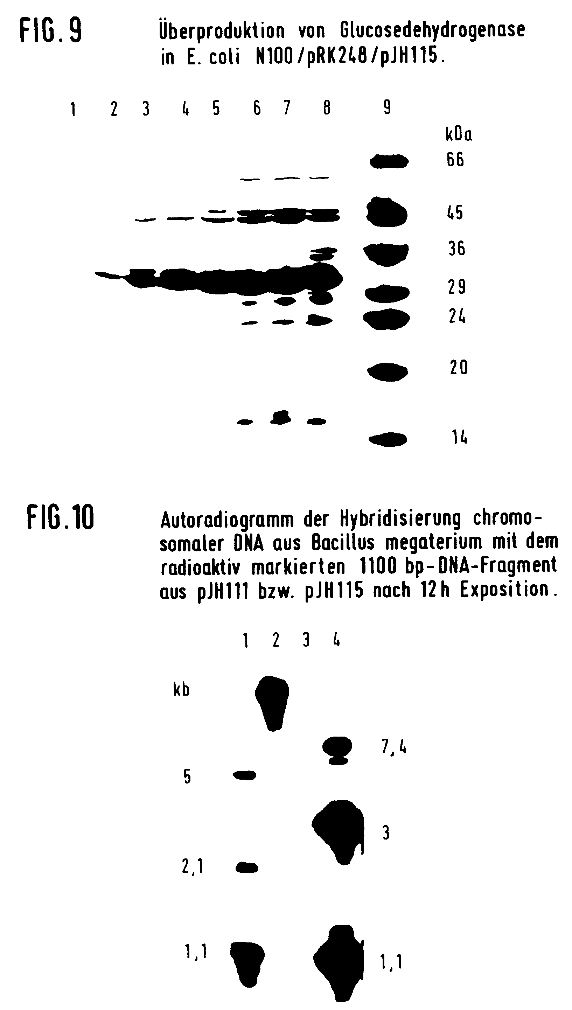

Neben der beträchtlichen Aktivitätssteigerung weist das erfindungsgemäße Verfahren aber auch noch weitere Vorteile auf. So entfallen alle Sporulationsprobleme in E. coli. Ein nach dem neuen Verfahren hergestellter Wirtsstamm läßt sich somit wesentlich einfacher und wirtschaftlicher verwenden. Ferner zeigen die Rohextrakte der nach dem neuen Verfahren Glucose-DH-produzierenden E. coli-Wirtszellen keinerlei Pigmentierung (klare, farblose Flüssigkeit), während die Rohextrakte, hergestellt nach konventioneller Art, stark dunkelgrün bis schwarz gefärbt sind und einer weiteren Aufreinigung bedürfen. Ein nach dem neuen gentechnologischen Verfahren hergestellter Rohextrakt enthält ferner keine nennenswert störenden $NADH_2$-Oxidasen. Die Reinheit bezüglich Fremdproteingehalt ist aus Figur 9 abzulesen. Die die rekombinante DNA enthaltenden Wirtszellen weisen weiterhin eine erstaunlich hohe Plasmidstabilität auf; außerdem zeigen die jeweiligen Expressionsprodukte eine gute Enzymstabilität, die für diagnostische Zwecke gewährleistet sein muß. Hier ist ein weiterer deutlicher Vorteil gegenüber dem bekannten Bacillus subtilis/E. coli-System festzustellen.

Das erfindungsgemäße Verfahren weist also entscheidende Vorteile gegenüber dem Stand der Technik auf.

Gegenstand der Erfindung ist somit ein Verfahren zur Herstellung von Glucose-DH durch Kultivierung eines mit rekombinanter DNA transformierten mikrobiellen Wirtsorganismus in einem Nährmedium und Isolierung der zur Expression gebrachten verschiedenen Polypeptide, das sich dadurch kennzeichnen läßt, daß ein Wirtsorganismus eingesetzt wird, der eine aus dem Genom von Bacillus megaterium isolierte DNA-Sequenz gemäß Fig. 1 enthält, die für ein Polypeptid mit der biologischen Aktivität des Enzyms Glucose-DH und der Aminosäure-Sequenz gemäß Fig. 2 codiert.

Gegenstand der Erfindung ist ebenfalls die DNA-Sequenz gemäß Figur 1, die für ein Polypeptid mit der biologischen Aktivität des Enzyms Glucosedehydrogenase codiert.

Gegenstand der Erfindung ist außerdem das Plasmid pJH111, welches die für das Glucose-DH-Polypeptid codierende erfindungsgemäße DNA-Sequenz enthält.

Gegenstand der Erfindung ist ferner ein rekombinantes Expressionsplasmid, in dem der gesamte codierende Bereich des Glucose-DH-Gens unter Kontrolle des $\lambda$-$P_L$-Promotors steht und die gesamte DNA-Sequenz des Glucose-DH-Gens bestimmt. Erfindungsgemäß können anstelle des $\lambda$-$P_L$-Promotors als Expressions-Kontroll-Sequenz ebenso ein E. coli Promotor-System wie das E. coli lac-System, das E. coli Lactamasesystem, das E. coli trp-System oder der E. coli Lipoprotein-Promotor, eine Hefeexpressions-Kontroll-Sequenz oder eine andere eukaryotische Expressions-Kontroll-Sequenz verwendet werden. Wichtig ist dabei nur die funktionelle Verknüpfung des Gens mit der Expressions-Kontroll-Sequenz sowie die Auswahl einer geeigneten Expressions-Kontroll-Sequenz für einen bestimmten Wirtsorganismus.

Gegenstand der Erfindung ist insbesondere das Expressionsplasmid pJH115, welches die für das Glucose-DH-Polypeptid codierende erfindungsgemäße DNA-Sequenz einschließlich des $\lambda$-$P_L$-Promotors enthält.

Gegenstand der Erfindung ist außerdem das die biologische Aktivität der Glucose-DH aufweisende Polypeptid mit der in Figur 2 angegebenen Aminosäuresequenz, das bei der Expression unter den genannten Bedingungen synthetisiert werden kann. Gegenstand der Erfindung ist auch der die erfindungsgemäße DNA enthaltende Wirtsorganismus, insbesondere E. coli N100/pRK248/pJH115, in welchen das Plasmid pJH115 transformiert wurde.

Gegenstand der Erfindung ist letztlich die Verwendung des erfindungsgemäßen Polypeptids zur Glucose-, insbesondere Blutzuckerbestimmung.

Erfindungsgemäß kann unter Verwendung der isolierten Glucose-DH-codierenden DNA-Sequenz aus Bacillus megaterium als Sondenmolekül in Genbanken Glucose-DH bildender Mikroorganismen das entsprechende Gen identifiziert und aus diesen Genbanken isoliert werden. Auf diese Weise ist auch die Herstellung von Polypeptiden mit der biologischen Aktivität des Enzyms Glucose-DH von anderen Mikroorganismen, z.B. Bacillus subtilis, in beliebiger Menge möglich.

Als Glucose-DH liefernde Spezies dienen Mikroorganismen der Art Bacillus megaterium sowie deren Mutanten oder Varianten, welche die entsprechende genetische Information besitzen. Stämme dieser Art sind bekannt und bei autorisierten Hinterlegungsstellen hinterlegt, z.B. DSM 321, DSM 322, DSM 333 oder DSM 337.

Als Wirtsorganismen, die mit Glucose-DH codierende DNA transformiert werden können, kommen in erster Linie Mikroorganismen, aber auch pflanzliche, tierische oder menschliche Zellen in Betracht. Vorzugsweise werden aber Mikroorganismen wie Bakterien, Hefen und andere Pilze, insbesondere E. coli-Bakterien, eingesetzt.

Das erfindungsgemäße Verfahren wird bevorzugt wie folgt durchgeführt.

Zunächst wird chromosomale DNA aus Bacillus megaterium-Zellen (Anzucht siehe Beispiel 1), mit Hilfe literaturbekannter Methodik isoliert und aufgereinigt (Beispiel 4). Die chromosomale aus Bacillus megaterium isolierte DNA wird mit Hilfe von hierfür geeigneten üblichen und bekannten, in der Regel käuflichen Restriktionsendonucleasen in an sich bekannter Weise partiell hydrolysiert. Vorzugsweise wird hierfür das Restriktionsenzym Sau3A eingesetzt. Die Hydrolyse wird so gesteuert, daß hauptsächlich Fragmente mit einer Länge zwischen 9 und 22 Kilobasen (kb), vorzugsweise mehr als 14 kb erhalten werden, da diese sich besonders vorteilhaft zum Einbau in die DNA eines Phagenvektors eignen. Ziel ist es, zur Klonierung des Strukturgens der Glucose-DH eine Genbank des entsprechenden Bacillus-Stammes mit Hilfe eines geeigneten Phagenvektors anzulegen. Hierfür dient vorzugsweise der bekannte Phagenvektor λ-EMBL3 (Frischauff et al., 1983, J. Mol. Biol. 170, 827-842). Die DNA des Phagen wird wie in Beispiel 3 näher beschrieben, präpariert und nach bekannter Weise mittels üblicher Restriktionsendonucleasen, vorzugsweise BamHI, aufgeschnitten. Die Größe der jeweilig erhaltenen DNA-Fragmente wird mittels Gelelektrophorese bestimmt.

In die Schnittstelle der Phagen-DNA werden nun die passenden DNA-Fragmente aus dem Bacillus-Genom mittels käuflicher Ligierungsenzyme, vorzugsweise $T_4$-DNA-Ligase, in an sich bekannter Weise eingebaut (Beispiel 5). Die so erhaltenen verschiedenen neukombinierten Phagen-Moleküle werden in einem zu ihrer Vermehrung befähigten Wirtsorganismus, vorzugsweise E. coli, insbesondere E. coli NM539-(Sup F hsd R lac Y $P_2$COx3) (ATCC 35 639) transduziert und in bekannterweise kloniert (Arber et al. 1983, in "Lambda II", Cold Spring Harbor Monographs, 433-465). Die Transformation von E. coli-Stammen allgemein wird z.B. nach der Calciumchlorid-Methode vorgenommen und ist im Beispiel 6 näher erläutert. Die Identifizierung des Glucose-DH-Gens erfolgt durch Nachweis der Enzymaktivität im Lysat der neuentstandenen Phagenklone mit Hilfe eines spezifischen Filtertests auf Basis der eingangs beschriebenen Glucose-DH-Nachweisreaktion. Die Bildung von $NADH_2$, die mit der Glucose-DH-Bildung einhergeht (positive Reaktion), wird durch das Auftreten von Fluoreszenzsignalen der auf Filterpapier aufgebrachten durch Phagen lysierten Zellen (Plaques) nachgewiesen (Beispiel 9). Aufgrund dieser ggf. zu wiederholenden Tests, die auch in den folgenden Arbeitsschritten zur Anwendung kommen, können schließlich Phagenklone mit reproduzierbarer positiver Reaktion identifiziert werden. Zur Bestätigung des Ergebnisses wird Flüssiglysat des isolierten Klons spektralphotometrisch (334, 340 oder 365 nm HG) untersucht und die Substratspezifizität gestestet. Dabei werden die aus der Literatur bekannten Daten erhalten (Pauly et al., 1975; Hoppe-Seyler's Z. Physiol. Chem. 356; 1613-1623).

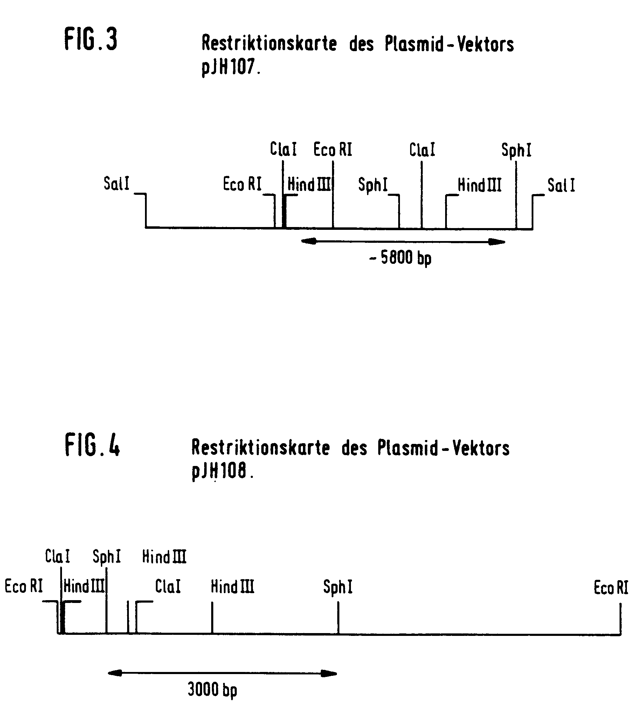

Um eine Konzentrierung der Glucose-DH-DNA innerhalb der rekombinanten Phagen-DNA zu erreichen, erfolgt in einem weiteren Arbeitsgang die Subklonierung vorzugsweise in Plasmiden. Hierzu wird wiederum die rekombinante Phagen-DNA partiell mit Restriktionsendonucleasen, vorzugsweise Sau3A, wie üblich hydrolysiert, so daß Fragmente von etwa 4-9 kb Größe entstehen. Als Ausgangsplasmid kann im Prinzip jedes Plasmid verwendet werden, welches bei einem Einbau von DNA-Fragmenten der angegebenen Größe nicht destabilisiert wird. Besonders hierfür geeignet ist das bekannte Plasmid pBR322 (Herkunft: Bolivar et al., 1977, Gene 2, 95-113), welches vorzugsweise mit der Restriktionsendonuclease BamHI anverdaut wird. Die Phagen-DNA-Fragmente werden nun in an sich bekannter Weise mit dem aufgeschnittenen Plasmid-Vektor ligiert (Beispiel 5). Mit den Ligierungsprodukten wird ein beliebiger, zur Transformation geeigneter und in einer anerkannten Hinterlegungsstelle erhältlicher Wirtsorganismus, vorzugsweise E. coli in üblicher Weise transformiert (Beispiel 6). Die so erhaltenen Kolonien werden dem Glucose-DH-Enzymtest (Beispiel 9) unterworfen. Dabei zeigen mehrere Klone eine positive Glucose-DH-Reaktion. Eines der zugrundeliegende Plasmide besitzt eine Größe von 10.2 kb und wird mit pJH 107 bezeichnet. Zum Zwecke der Restriktionsanalyse wird das Plasmid in an sich bekannter Weise durch die Restriktionsendonucleasen EcoRI, SalI, SphI, HindIII und ClaI vollständig hydrolysiert, und die DNA-Fragmente werden mittels einer Agarosegelelektrophorese in an sich bekannter Weise aufgetrennt. Die Größe des Passagier-Fragmentes, welches die Glucose-DH-Sequenz enthält, wird mit 5800 bp ermittelt. Figur 3 gibt die Restriktionskarte des erfindungsgemäßen Plasmids pJH107 wieder.

Zur weiteren Eingrenzung des Gens auf dem rekombinanten Plasmid wird in folgender Weise vorgegangen, wobei die Verfahrensweise oben und in den Beispielen beschrieben oder allgemeiner Stand der Technik ist.

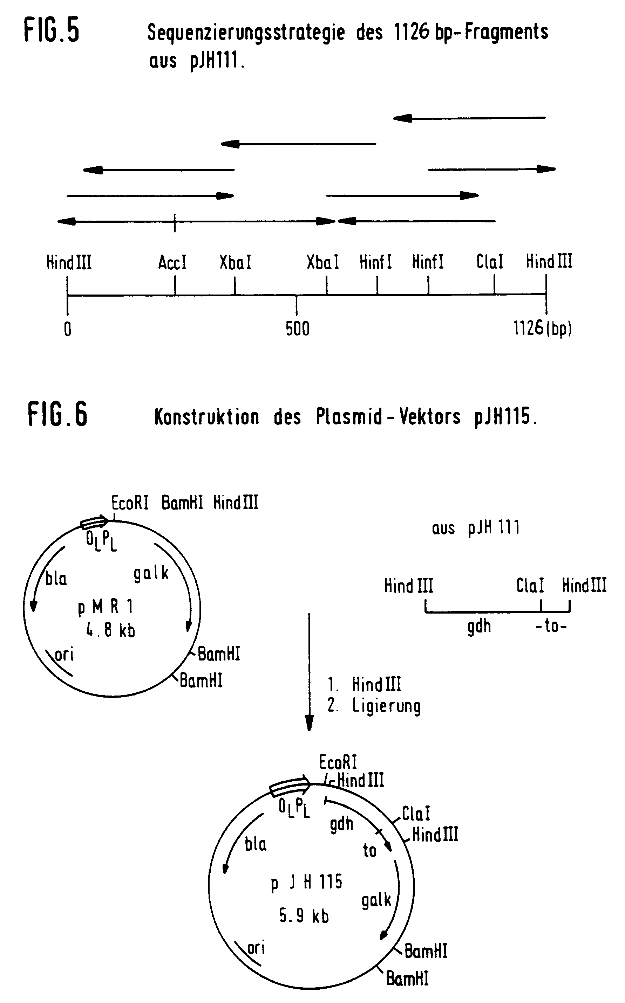

- Verdauung von pJH107 mit Restriktionsendonucleasen, die mit hoher Wahrscheinlichkeit nicht im Strukturgen schneiden. Vorzugsweise kommt hier SphI zum Einsatz.
- Einbau in den Plasmid-Vektor pBR322 und Transformation und Klonierung eines Wirtsorganismus, vorzugsweise E. coli RR1. Neukombinierter erfindungsgemäßer Plasmid-Vektor: pJH 108, enthält ein ca. 3000 bp großes Passagier-DNA-Fragment (Figur 4). Bei der Restriktionsanalyse werden vorzugsweise die Endonucleasen, SphI, ClaI, EcoRI und HindIII eingesetzt.
- Verdauung von pJH108, vorzugsweise mit HindIII.
- Einbau in den Plasmid-Vektor pBR322 und Transformation und Klonierung eines Wirtes, vorzugsweise E. coli. Neukombinierter erfindungsgemäßer Plasmid-Vektor: pJH111 (Figur 5).

Der Plasmid-Vektor pJH111 enthält wie die Restriktionsanalyse zeigt, nur noch ein 1100 bp großes Passagier-DNA-Fragment neben dem 5500 bp goßen Rest-Plasmid. Die entsprechenden E. coli-Klone zeigen Glucose-DH-Enzymaktivität. Eine weitere Verkleinerung des DNA-Fragmentes führt zu vollständigem Verlust der Enzymaktivität. Das Gen für die Glucose-Dehydrogenase ist also auf dem 1100 bp-Fragment lokalisiert.

Die Bestimmung der Nukleotidsequenz des Glucose-DH-Gens erfolgt beispielsweise nach der Methode von Maxam und Gilbert (1980, Methods Enzymol., 65, 499-580). Bei dieser Methode wird radioaktiv markierte DNA in vier verschiedenen basenspezifischen Reaktionen partiell gespalten, die Spaltprodukte werden auf einem denaturierten Polyacrylamidgel getrennt und die Sequenz nach anschließender Autoradiographie in an sich bekannter Weise ermittelt (Beispiel 7).

Die Sequenzierungsstrategie ist in Figur 5 abgebildet. Die dem Strukturgen zuzuordnende neue erfindungsgemäße Nukleotidsequenz ist in Figur 1 wiedergegeben.

Figur 2 zeigt die aus der DNS-Sequenz abgeleitete neue Aminosäuresequenz. Sie entspricht derjenigen, die durch Protein-Sequenzierung des aufgereinigten Enzyms erhalten wird.

Die Expression des erfindungsgemäßen Polypeptids gemäß Figur 2 in einem E. coli-Wirt unter Benutzung des Plasmidvektors pJH111, aber ohne einen spezifischen E. coli-Promotor führt, wie oben angegeben, zu Enzymaktivitäten von durchschnittlich etwa 0,1 Units/ml Kultur. Um zu einer gewünschten Überproduktion zu kommen, wird ein Expressionvektor benutzt. Als Expressionsvektor kommt beispielsweise pMR1 (Herkunft: Rimmler, 1985, Dissertation, TH Darmstadt) in Frage. Dieses Plasmid, welches aus den literaturbekannten Plasmiden pDS26t und pPlc28 konstruierbar ist, enthält den effizienten λ-$P_L$-Promotor. Das Plasmid pPlc28 sowie das zur Klonieung verwendete Wirtsbakterium E. coli W6 sind aus der europäischen Patentanmeldung EP 00 41 767 bekannt. In E. coli W6 ist der λ-$P_L$-Promotor durch den chromosomal codierten cl-Repressor reprimiert. pJH111 wird mit HindIII geschnitten und das erfindungsgemäße 1100 bp-Fragment wird nach präparativer Gelelektrophorese über "low melting" Agarose in an sich bekannter Weise isoliert. pMR1 wird ebenfalls mit vorzugsweise HindIII hydrolysiert und die Spaltprodukte werden in üblicher Weise ligiert und in E. coli W6 transformiert (Remaut et al., 1981, Gene 15, 81-93). Die Plasmid-DNA wird, wie angegeben (Beispiel 3) isoliert und ein beliebiger zur Transformation geeigneter, in einer anerkannten Hinterlegungsstelle erhältlicher E. coli-Stamm, in den in bekannter Weise das Plasmid pRK248 (Herkunft: Bernard et al. 1979, Gene 5, 59-76) einkloniert wurde, transformiert. Letzteres trägt das Gen für den temperatursensitiven λ-Repressor und für die Tetracyclinresistenz. Das neukonstruierte erfindungsgemäße Plasmid trägt die Bezeichnung pJH115. Seine Konstruktion ist in Figur 6 wiedergegeben. Der erfindungsgemäße Wirtsorganismus trägt die Bezeichnung E. coli N100/pRK248/pJH115. Erfindungsgemäß kann auf die Anwesenheit von pRK248 verzichtet werden, wenn Wirtsorganismen herangezogen werden können, bei denen der λ-Repressor chromosomal codiert wird (z.B. E. coli W6). Der erfindungsgemäße Wirtsorganismus zeigt die oben und in Figur 8 angegebenen überraschend hohen Expressionen. Ein typisches Expressionsexperiment ist in Beispiel 10 dargelegt.

Die Unterschiede der für Glucose-DH codierenden erfindungsgemäßen DNA- bzw. der daraus abgeleiteten Aminosäuresequenz im Vergleich zur bekannten Glucose-DH legen den Schluß nahe, daß neben dem neuen erfindungsgemäßen Enzym noch ein zweites Enzym bzw. Gen in Bacillus megaterium existiert. Die Identifizierung von Isoenzymen der Glucose-DH erfolgt durch Hybridisierung filtergebundener DNA aus Bacillus megaterium mit dem radioaktiv markierten 1126 bp HindIII-Fragment des Gens (Beispiele 7 und 8). Zu diesem Zweck wird in an sich bekannter Weise chromosomale DNA mit der Restriktionsendonuklease HindIII hydrolysiert, gemeinsam mit DNA-Längenstandards auf ein 1 % Agarosegel aufgebracht und auf Nitrozellulose übertragen. Als Längenstandard dient neben der durch Eco RI hydrolysierten SPP1 DNA ein Gemisch aus pJH108 (Sal I-Sph I hydrolysiert) und pJH111 (HindIII hydrolysiert), wodurch eine Größenbestimmung hybridisierender Fragmente nach der Autoradiographie ermöglicht wird. Hybridisierungstechnik und Autoradiographie sind, sofern nicht in den Beispielen näher erläutert, Stand der Technik bzw. für den Fachmann in einfacher Weise daraus ableitbar. Aus dem Autoradiogramm (Figur 10), können neben der der eingesetzten Gensonde entsprechenden 1100 bp intensiven Bande schwächere Banden bei 2100 und 5000

6

bp identifiziert werden. Damit ist gezeigt, daß Bacillus megaterium mehr als eine Glucose-Dehydrogenase enthält.

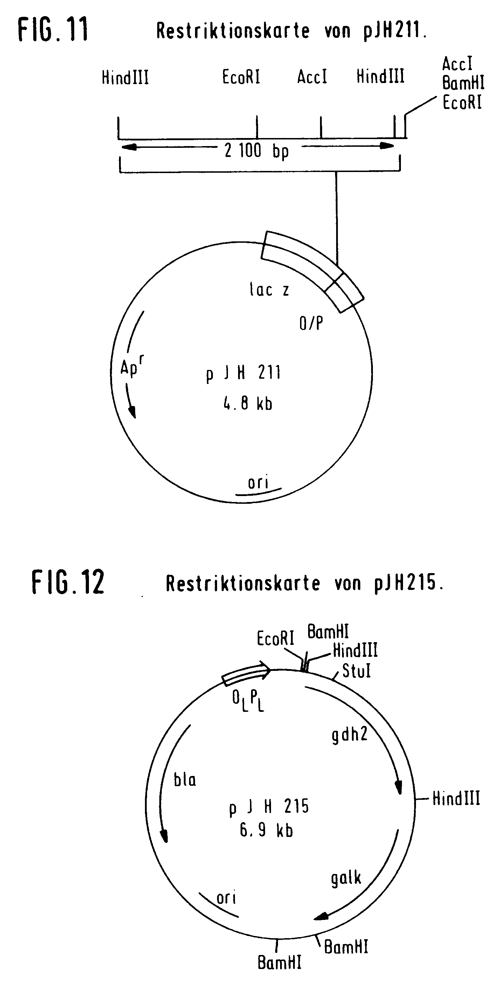

Die Klonierung einer weiteren Glucose-DH entsprechend der Fragmente zwischen 1300 und 5000 bp kann prinzipiell wie oben für das 1100 bp-Fragment ausführlich beschrieben durchgeführt werden. Als Vektor dient das E. coli-Plasmid pUC18 (Herkunft: Viera et al., 1982, Gene 19, 259). Man erhält schließlich das neue Plasmid pJH211 (Figur 11) welches ein 2100 bp DNA-Fragment enthält und das in E. coli zur Expression von Glucose-DH führt (das gleichfalls mit der Gensonde hybridisierende 5000 bp-Fragment kann hierbei nicht erhalten werden). In an sich bekannter Weise, bzw. analog zum oben beschriebenen 1100 bp-Fragment wird die DNA- bzw. Proteinsequenz des zweiten Glucose-DH-Gens ermittelt. Sie entspricht dabei vollkommen der bekannten Sequenz der Glucose-DH. In Analogie zum Glucose-DH produzierenden, erfindungsgemäßen E. coli-Stamm E. coli N100/pRK248/pJH115 kann ein entsprechender Überproduktions-Stamm fur die zweite Glucose-DH konstruiert werden: E. coli N100/pRK248/pJH215 liefert erfindungsgemäß in großen Mengen qualitativ hochwertige Glucose-DH, wobei die Enzymaktivität ca. 20-30 % niedriger ist als im System E. coli N100/pRK248/pJH115. Die Stabilität beider Isoenzyme ist zwischen 20 und 50° (Vorinkubation) annähernd vergleichbar.

In der Beschreibung, in den Beispielen und Figuren werden, sofern dort nicht näher erläutert, folgende Abkürzungen verwendet:

| | |
|---|---|
| A | Adenin |
| $A_{578}$ | Absorption bei 578 nm |
| $A_{578}$-E | Absorptionseinheit bei 578 nm |
| $Ap^R$ | Ampicillinresistenz |
| APS | Ammoniumperoxodisulfat |
| b | Base |
| Bis | N,N,N′,N′-Methylenbisacrylamid |
| bp | Basenpaar |
| BSA | Rinderserumalbumin |
| C | Cytosin |
| Ci | Curie (2.22 · $10^{12}$ Zerfälle pro Minute) |
| cpm | Zählimpulse pro Minute |
| Da | Dalton (g/mol) |
| DNA | Desoxyribonukleinsäure |
| DTT | Dithiothreitol |
| EDTA | Ethylendiamintetraessigsäure |
| E. coli | Escherichia coli |
| G | Guanin |
| galK | Galaktokinase |
| k | x $10^3$ |
| l | Liter |
| NAD | Nicotinamid-adenin-dinukleotid |
| NADP | Nicotinamid-adenin-dinukleotid-phosphat |
| PEG | Polyethylenglykol |
| rpm | Umdrehungen pro Minute |
| s | Sekunde |
| SDS | Natriumdodecylsulfat |
| T | Thymin |
| T4 | Bakteriophage T4 |
| $Tc^R$ | Tetracyclinresistenz |
| TEMED | N,N,N′,N′-Tetramethylethylendiamin |
| Tris | Tris-(hydroxymethyl)-aminomethan |
| tRNA | Transfer-Ribonukleinsäure |
| U | Einheit der Enzymaktivität (Unit) |

Im folgenden sind die Abbildungen erläutert, in denen einzelne Teilbereiche der Erfindung dargestellt sind:

Figur 1: DNA-Sequenz des Glucosedehydrogenase-Gens aus Bacillus megaterium

Figur 2: Aminosäure-Sequenz des Expressionsproduktes von pJH111 bzw. pJH115.

Figur 3: Restriktionskarte des Plasmid-Vektors pJH107. Eingezeichnet sind die eindeutig zugeordneten Restriktionsschnittstellen. Die Größe der Passagier-DNA beträgt ca. 5800 bp. Das Plasmid pJH107 ist konstruiert aus dem BamHI-verdauten Plasmid pBR322 und der Sau3A-

7

hydrolysierten Konstruktion aus chromosomaler DNA und λ-EMBL-3 Phagen-DNA.

Figur 4: Restriktionskarte des Plasmid-Vektors pJH108. Eingezeichnet sind nur die eindeutig zugeordneten Schnittstellen. Die Größe der Passagier-DNA beträgt ca. 3000 bp. Das Plasmid pJH108 ist konstruiert aus einem Fragment von pJH107 (SphI hydrolysiert) und pBR322.

Figur 5: Sequenzierungsstrategie des 1126 bp-Fragments aus pJH111. Die Pfeile geben die Größe der jeweils identifizierten Sequenz an. Das Plasmid pJH111 ist konstruiert aus dem HindIII-verdauten Fragment von pJH108 und pBR322.

Figur 6: Konstruktion des Plasmid-Vektors pJH115. Dieser ist konstruiert durch HindIII-Hydrolyse von pMR1 und pJH111 und anschließende Ligierung.

bla: Beta-Lactamase; gdh: Glucose-DH;
to: Terminator; galk: Galactokinase;
$O_L P_L$: Promotor-Operator-Region (λ-$P_L$-Promotor)
Ori: Replikationsursprung

Figur 7: Proteinsequenzvergleich zwischen Glucose-DH, basierend auf 1100 bp-Fragment (a), und Glucose-DH, basierend auf 2100 bp-Fragment (b; bekannte Sequenz). Dargestellt sind die ersten 45 Aminosäuren.

Figur 8: Wachstums- und Expressionskurve von E. coli N100/pRK248/pJH115. Bakterienwachstum in 20 ml LB-Medium (Beispiel 1) bei 28 °. Bei einer optischen Dichte von 0,5 $A_{578}$-E wurde die Kultur in ein 42 °-Wasserbad überführt, je 1 $A_{578}$-E Zellen entnommen und die Enzymaktivität bestimmt.

Figur 9: Überproduktion von Glucosedehydrogenase in E. coli N100/pRK248/pJH115. Proteintrennung auf einem 12,5 % SDS-Polyacrylamidgel

Spur 1: Zellprotein vor der Induktion
Spur 2-8: 0,5; 1-5 und 10 h nach Induktion
Spur 9: Größenstandard

Figur 10: Autoradiogramm der Hybridisierung chromosomaler DNA aus Bacillus megaterium mit dem radioaktiv markierten 1100 bp-DNA-Fragment aus pJH111 bzw. pJH115 nach 12 h Exposition.

Spur 1: Chromosomale DNA (Bacillus meg.), HindIII-hydrolysiert
Spur 2: Chromosomale DNA (Bacillus meg.), unhydrolysiert
Spur 3: SPP1-Längenstandard
Spur 4: Gemisch aus je 10 ng:
pJH108, SalI-hydrolysiert
pJH108, SphI-hydrolysiert
pJH111, HindIII-hydrolysiert

Figur 11: Restriktionskarte von pJH211. Nur die zur Restriktionsanalyse verwendeten Schnittstellen sind eingezeichnet. Das Plasmid pJH211 ist konstruiert aus pUC18 und HindIII-hydrolysierter chromosomaler DNA.

Figur 12: Restriktionskarte von pJH215. Das Plasmid pJH215 ist konstruiert aus pJH211 (HindIII-hydrolysiert) und pMR1.
Bezeichnung wie in Fig. 6.

Die folgenden Beispiele sollen die Erfindung näher erläutern. Die Temperaturen sind durchweg in Grad Celsius angegeben.

Beispiel 1 Anzucht von E. coli-Bakterien

a) Zur Präparation von Plasmiden in mg-Mengen erfolgt die Anzucht von E. coli-Bakterien im 1 l Maßstab in Voll-(LB) oder Minimalmedium (M9). Zu diesem Zweck wird 1 l Medium mit 5 ml einer stationären Vorkultur beimpft und unter ständigem Schütteln ca. 18 h bei 37 ° bis zum Erreichen der stationären Wachstumsphase kultiviert.

b) Für die Überexpression der Glucose-Dehydrogenase wird 200 ml LB-Medium mit 2 ml einer bei 28 ° gewachsenen Übernachtkultur beimpft. Die Bakteriensuspension wird daraufhin bei 28 ° und 160 rpm bis zum Erreichen einer optischen Dichte von 0,5 $A_{578}$-E kultiviert. Im Anschluß wird das Kulturgefäß weitere 16 h bei 42 ° inkubiert.

a) LB-Medium:

| Casein, enzymatisch hydrolysiert | 10 g |
|---|---|
| Hefeextrakt | 5 g |
| NaCl | 5 g |
| Agar (Festagar) | 15 g |
| (Weichagar) | 7,5 g |
| Aqua bidest. | ad 1000 ml |

pH-Wert mit 1,0 N NaOH auf 7,4 einstellen Antibiotika wurden in folgenden Konzentrationen zugesetzt:

| Ampicillin: | 50 $\mu$g/ml |
|---|---|
| Tetracyclin: | 20 $\mu$g/ml |

b) Modifiziertes M9-Medium:

| M9-Salze 10-fach konzentriert | 10 ml |
|---|---|
| 20 % Glucoselösung | 5 ml |
| 1 M $MgSO_4$ | 1 ml |
| 10 % Caseinhydrolysat | 5 ml |
| Thiaminlösung (2 mg/ml) | 0,1 ml |
| Aqua bidest. | ad 1000 ml |

c) M9-Salze 10-fach konzentriert:

| $Na_2HPO_4$ x 2 $H_2O$ | 75 g/l |
|---|---|
| $KH_2PO_4$ | 30 g/l |
| $NH_4Cl$ | 10 g/l |
| NaCl | 5 g/l |

Beispiel 2 Präparation von λ-Phagen

a) Plattenlysate

Um aus Phagensuspensionen mit niedrigem Titer eine größere Anzahl rekombinanter Phagen zu präparieren, werden zunächst Plattenlysate hergestellt.

Zu diesem Zweck werden $10^5$-$10^6$ Phagen in einem Volumen von 100 $\mu$l mit 100 $\mu$l einer Übernachtkultur des Wirtsstammes E. coli NM 539 versetzt und zur Phagenadsorption unbewegt bei 37° inkubiert. Im Anschluß werden 3 ml 47-50° heißer Weichagar zugegeben, gemischt und auf einer Festagarplatte verteilt. Nach 16stündiger Inkubation bei 37° wird die Weichagarschicht abgekratzt. Anschließend wird durch Zentrifugation (10 min) bei 10 000 rpm und 4° die Zelldebris abgetrennt. Der Überstand wird in ein steriles Gefäß überführt, mit einigen Tropfen Chloroform versetzt und bei 4° aufbewahrt.

b) Flüssiglysate

Die Gewinnung von Phagen in präparativem Maßstab erfolgt mit Hilfe von Flüssiglysaten.

25 ml LB-Flüssigmedium, welches 10 mM $MgSO_4$ und 0,4 % Maltose enthält, wird mit einer Übernachtkultur des Wirtsstammes E. coli NM 539 beimpft. Unter Belüftung wird die Suspension bis zum Erreichen von ca. 1,3 $A_{578}$-E bei 37° inkubiert. Im Anschluß werden die Bakterien mit $10^6$ Phagen/ml infiziert und zur Adsorption 15 min unbewegt bei 37° belassen. Mit den infizierten Bakterien wird daraufhin 1 l 37° warmes LB-Medium inokuliert und bis zum Eintreten der Lyse bei dieser Temperatur unter Belüftung inkubiert. Das Lyseereignis läßt sich durch einen rapiden Abfall der Extinktion bei 578 nm spektralphotometrisch verfolgen. Nach Entfernen der Zelldebris durch Zentrifugation für 15 min bei 5000 rpm und 4° können die Phagen im wäßrigen Überstand erhalten werden.

Beispiel 3 Isolierung von Vektor-DNA

a) Isolierung von Phagen-DNA

Die Suspension von durch Ultrazentrifugation gereinigten Phagen wird auf 20 mM EDTA, 0,5 % SDS eingestellt und nach Zugabe von 50 μg/ml Proteinase K 1 h bei 50° inkubiert. Anschließend wird mehrfach mit gleichem Volumen Phenol und mehrfach mit Chloroform/Isoamylalkohol (24:1) extrahiert. Die DNA wird mit Ethanol in bekannter Weise gefällt und durch Zentrifugation 10 min bei 5 000 rpm sedimentiert. Daraufhin wird sie mit 70 % Ethanol gewaschen, getrocknet und in Puffer aufgenommen.

b) Minipräparation von Plasmiden

Um Plasmide in großer Zahl analysieren zu können, wird die Kurzlysatmethode nach Birnboim (Birnboim und Doly, 1979: Nucl. acid Res. 7, 1513) verwendet.

Je 1,5 ml Zellsuspension aus einer Übernachtkultur wird durch Zentrifugation 1 min bei 15 000 rpm sedimentiert. Nach Zugabe von 100 μl Lösung A (siehe unten) wird 5 min bei 20° inkubiert. Anschließend werden 200 μl Lösung B (siehe unten) zugegeben und die Gefäße werden in Eis gestellt. Nach weiteren 5 min wird Lösung C (siehe unten) zugesetzt und die Proben werden solange in Eis belassen, bis ein unlöslicher Komplex aus chromosomaler DNA, RNA und Protein ausfällt (2-5 min). Nach einer Zentrifugation (15 min) werden die plasmidhaltigen Überstände in neue 1,5 ml-Eppendorf-Kunststoffgefäße überführt.

Die DNA wird mit Ethanol 10 min bei -20° gefällt und 15 min in der Zentrifuge sedimentiert. Im Anschluß wird sie zweimal mit 70 % Ethanol gespült und (im Exsikkator) getrocknet.

Nach Aufnahme in 40 μl TE-Puffer (siehe unten) kann sie direkt für die Transformation oder für Restriktionsanalysen eingesetzt werden:

```
Birnboim-Lösung A:              Birnboim-Lösung B:


25 mM Tris-HCl, pH 8,0          0,2 M NaOH
50 mM Glucose                   1   % SDS
10 mM EDTA


Birnboim-Lösung C:              TE-Puffer:


3 M NaOAc/HOAc, pH 4,8          10 mM Tris-Base
                                 1 mM HCl
                                pH: 8,0
```

c) Präparative Plasmidisolierung

Die Präparation von Plasmiden in mg-Mengen erfolgt mit Hilfe einer limitierten Protoplasten-Lyse durch Detergenzien (Hardies et al., 1979, J. Biol.Chem. 254 5527-5534).

Eine Bakteriensuspension nach Beispiel 1 wird 20 min bei 5 000 rpm und 4° zentrifugiert. Das Sediment wird in 15 ml Saccharoselösung (25 % Saccharose in 50 mM Tris-HCl, pH 8.0) aufgenommen. Nach Zugabe von 3 ml 0,5 M EDTA und 3 ml Lysozymlösung (20 mg/ml Lysozym in 50 mM Tris-HCl, pH 8,0) wird 30-40 min in Eis inkubiert. Danach werden 2 ml einer 2:1 (v/v) Mischung aus 10 % Polyethylenglycolmonolaurylether/10 % Desoxycholat zugegeben. Die Klärung des Zellaufschlusses erfolgt durch Zentrifugation (30 min) bei 40 000 rpm und 4°. Der klare Überstand wird dekantiert, 100 μg/ml RNase A zugegeben und 45 min in Eis inkubiert. Zur DNA-Fällung wird der RNase-behandelte Überstand mit einem halben Volumen 30 % PEG 6 000 in 1,5 M NaCl versetzt und weitere 30 min in Eis belassen. Man zentrifugiert 20 min bei 8 000 rpm und 4°. Der Niederschlag wird in 5 ml TE-Puffer aufgenommen, 50 μg/ml Proteinase K zugegeben und für 60 min bei 37° inkubiert. Danach wird mehrfach mit TE-gesättigtem Phenol und mehrfach mit Chloroform/Isoamylalkohol (24:1) extrahiert. Die DNA wird mit Ethanol gefällt, gewaschen und getrocknet. Das Sediment wird in 1-2 ml TE-Puffer gelöst und die Plasmidausbeute spektralphotometrisch bei 260 nm bestimmt.

Beispiel 4: Präparation chromosomaler DNA aus Glucose-DH-bildenden Bacillus megaterium-Zellen

Bakterienzellen aus 100 ml einer in Vollmedium gewachsenen stationären Kultur werden 10 min bei 5 000 rpm und 4° sedimentiert. Anschließend wird das Sediment in 10 ml Lysepuffer (50 mM NaCl, 50 mM EDTA, 30 mM Tris-HCl, pH 7.9) aufgenommen; 40 mg Lysozym werden zugegeben und 45 min bei 37° inkubiert. Die Suspension wird auf 1 % SDS eingestellt, mit 5 mg Proteinase K versetzt und weitere 30 min bei 37° belassen. Danach wird mehrfach mit TEgesättigtem Phenol und mehrfach mit Chloroform/Isoamylalkohol (24:1) extrahiert. Die Nukleinsäuren werden in üblicher Weise gefällt, gewaschen und getrocknet.

Zur Hydrolyse noch vorhandener RNA wird das Sediment vorsichtig in 5 ml TE-Puffer resuspendiert, 10 $\mu$g/ml RNase zugefügt und 30 min bei 37° inkubiert.

Die Lösung wird daraufhin auf 1 % SDS und 50 $\mu$g/ml Proteinase K eingestellt und weitere 30 min bei 37° behandelt. Anschließend wird wie oben beschrieben mit Phenol und Chloroform/Isoamylalkohol extrahiert und gegen TE-Puffer dialysiert. Die Ausbeute an chromosomaler DNA beträgt bei dieser Methode zwischen 2 und 4 mg.

Beispiel 5: Ligierung von DNA

Die Bildung von Phosphodiesterbindungen zwischen benachbarten $3'$-Hydroxyl- und $5'$-Phosphatgruppen wird durch T4-DNA-Ligase katalysiert.

Die Reaktion erfolgt in T4-DNA-Ligasepuffer mit einem 5fachen Überschuß der zu inserierenden DNA gegenüber der Vektor-DNA. Das Volumen beträgt 20 $\mu$l.

Bei DNA-Fragmenten mit einzelsträngig überstehenden Enden wird die Ligierung 4-16 h bei 4° mit 1 U Enzym pro $\mu$g DNA durchgeführt. Die Ligierung von doppelsträngigen Enden erfolgt 4 h bei 20° mit 10 U Enzym pro $\mu$g DNA.

Anschließend wird das Reaktionsgemisch direkt für die Transformation eingesetzt (Beispiel 6).

T4-DNA-Ligasepuffer:

0,4 mM ATP
66 mM Tris-HCl, pH 7.6
6,6 mM $MgCl_2$
10 mM DTT

Beispiel 6: Transformation von E. coli (Calciumchlorid-Methode)

a) Herstellung kompetenter E. coli-Zellen
E. coli Zellen werden in 20 ml LB-Medium bis zum Erreichen einer optischen Dichte von ca. 0,5 $A_{578}$-E kultiviert. Nach dem Überführen in ein vorgekühltes Zentrifugenröhrchen wird die Suspension 10 min in Eis gestellt. Die Bakterien werden 10 min bei 8 000 rpm und 4° sedimentiert. Nach dem Dekantieren des Überstandes werden die Zellen mit 20 ml eiskalter 0,1 M $MgCl_2$-Lösung gewaschen und, wie beschrieben, zentrifugiert. Im Anschluß daran werden sie in 1 ml eiskalter 0,1 M $CaCl_2$-Lösung aufgenommen und mindestens 2 h in Eis belassen.
b) Transformation kompetenter E. coli-Zellen
Zur Transformation werden 200 $\mu$l kompetente Zellen mit 50 ng Plasmid-DNA versetzt und 30 min in Eis gestellt. Daraufhin wird der Ansatz 2 min im Wasserbad (42°) erhitzt (Hitzepuls). Nach Zugabe von 1 ml LB-Medium wird 1 h bei 37° inkubiert (Erholungsphase).

Je 200 $\mu$l dieser Bakteriensuspension werden im Anschluß auf Selektivnährböden ausplattiert. Die Platten werden 16-24 h bei 37° bebrütet.

Für die Transformation des temperatursensitiven E. coli-Stammes N 100-pRK 248 clts werden alle Inkubationsschritte bei 28° durchgeführt. Der Hitzepuls erfolgt 5 min bei 34°.

Beispiel 7: Radioaktive Markierung von DNA

Radioaktiv markierte DNA-Fragmente mit einer spezifischen Aktivität > $10^8$ cpm/$\mu$g werden mit Hilfe der E. coli-DNA-Polymerase I durch Reparatursynthese ("nick translation") in Gegenwart von $\alpha^{32}$P-Desoxynukleotiden dargestellt.

Ansatz:

0,2 µg DNA
2 µl 10 x DNA Polymerase-Puffer
je 1 µl 1,2 mM dNTPs (Nukleotide 1-3)
5 µl $\alpha^{32}$P-dNTP (3 000 Ci/mmol, 10 µCi/µl) (Nukleotid 4)
0,5 µl DNase I (60 pg/µl)
1 min bei 20° reagieren lassen und Zugabe von
10 U DNA Polymerase I

10 x DNA Polymerase-Puffer:

500 mM Tris-HCl, pH 7,8
50 mM $MgCl_2$
100 mM $\beta$-Mercaptoethanol
0,05 BSA

Die Reaktion erfolgt nach Zugabe von DNA-Polymerase I 2-4 h bei 14° und wird mit 20 µl 60 mM EDTA, pH 8,0 gestoppt. Im Anschluß wird das Volumen mit $H_2O$ auf 100 µl erhöht.

Zur Entfernung von nicht umgesetzten Nukleotiden werden die Proben mit 100 µl 5 M Ammoniumacetatlösung und 400 µl absolutem Ethanol versetzt, gemischt und 2 min in flussigem Stickstoff eingefroren. Die radioaktiv markierten DNA-Fragmente werden daraufhin 15 min in einer Tischzentrifuge sedimentiert, mit 70 % Ethanol gespült und getrocknet. Nicht umgesetzte Nukleotide bleiben im Überstand.

Die DNA wird in 100 µl $H_2O$ aufgenommen und in der Folge für Hybridisierungsexperimente verwendet.

Beispiel 8: Hybridisierung filtergebundener DNA

Die Hybridisierung filtergebundener DNA erfolgt in Lösungen mit 50 % Formamid. Zur Abdeckung unspezifischer Bindestellen werden die mit DNA-Fragmenten beschichteten Nitrozellulosefilter zusammen mit der sie bedeckenden Prähybridisierungslösung luftblasenfrei in Plastikfolie eingeschweißt und 3 h bei 42° inkubiert.

Prähybridisierungslösung:

50 % Formamid
5fach konzentrierte Denhardts Lösung
5fach konzentriertes SSC (siehe unten)
0,5 % SDS
100 µg/ml tRNA aus Hefe

Denhardts Lösung:

0,02 % BSA
0,02 % Polyvinylpyrrolidon
0,02 % Ficoll (Polysucrose)

SSC:

150 mM NaCl
15 mM Natriumcitrat, pH 7.2

Zur Hybridisierung wird zunächst die radioaktiv markierte, doppelsträngige DNA-Sonde durch 10 minütiges Erhitzen auf 80° denaturiert und sofort in Eis abgekühlt.

Danach werden die nun einzelsträngig vorliegenden Nukleinsäuren in 20-30 ml Hybridisierungslösung übergeführt. Mit dieser wird die Prähybridisierungslösung ersetzt und weitere 20 h bei 42° inkubiert.

Hybridisierungslösung:

50 % Formamid
5fach konzentrierte Denhardts Lösung

5fach konzentriertes SSC
0,5 % SDS
10 mM EDTA
100 $\mu$g/ml tRNA aus Hefe
$10^6$ cpm/ml radioaktiv markierte DNA
Zur Entfernung unspezifisch gebundener DNA werden die Filter nach der Hybridisierung zunächst mehrfach mit 200 ml 2fach konzentrierte SSC, 0,1 % SDS bei 20° und im Anschluß mehrfach mit 10 %igem SSC, 0,1 % SDS bei 42° gewaschen.

Beispiel 9: Enzymtest für die Glucose-DH

Für diesen Test wird nicht-fluoreszierendes Chromatographiepapier mit 92 ml Indikatorlösung gleichmäßig besprüht und im Anschluß bei 20° getrocknet.

Indikatorlösung:

60,5 ml Natriumphosphat-Puffer (0,12 M; pH 7,6)
30 ml D-Glucose-Lösung (10 % w/v)
1,5 ml NAD$^+$-Lösung (70 mg/ml)
Das präparierte Chromatographiepapier wird vor der Durchführung des Enzymtests auf die Größe der Nährbodenplatten zurechtgeschnitten (Durchmesser ca. 8 cm).

Zur Durchführung des Tests bei Phagenlysaten werden die Platten sofort nach Bildung gut sichtbarer Plaques (600-800 pro Platte) dem Brutschrank entnommen. Zunächst werden die Petrischalen markiert und identisch gekennzeichnete Testfilter deckungsgleich aufgelegt. Nach 2 min werden sie abgenommen, im Heißluftstrom getrocknet und im Anschluß unter Licht der Wellenlänge 366 nm betrachtet. Wurde aktive Glucose-DH auf die Test-Filter übertragen, so läßt sich an diesen Stellen NAD$^+$ zu NADH reduzieren, welches durch Fluoreszenz deutlich wahrgenommen werden kann. Mit Hilfe der Filtermarkierungen werden daraufhin positive Phagenklone auf der Nährbodenplatte identifiziert und isoliert.

Zur Durchführung des Tests bei Bakterienklonen werden Zellen in identischer Weise auf zwei Nährbodenplatten übertragen (100-200 Kolonien pro Platte). Während eine der Platten als Referenzplatte dient ("master plate"), wird die andere für den Enzymtest verwendet. Dabei werden die Bakterien auf Aluminiumfolie transferiert und ca. 5 $\mu$l der Aufschlußlösung auf jede Kolonie pipetiert. Zur Lyse werden die Zellen 30 min bei 20° in einer feuchten Kammer inkubiert. Anschließend wird, wie oben beschrieben, ein Testfilter aufgelegt, getrocknet und die Stelle der enzymatischen Reaktion identifiziert. Die entsprechenden positiven Bakterien der Referenzplatte können im Anschluß zur Plasmidisolierung (Beispiel 3) verwendet werden.

Aufschlußlösung:

100 mM Natriumphosphat-Puffer, pH 6.5
20 mM EDTA
5 mg/ml Lysozym
Die Aktivitätsbestimmung erfolgt am Spektralphotometer bei 25°.

Testlösung:

120 mM Natriumphosphat-Puffer, pH 7.6
100 mM Glucose
2 mM NAD$^+$
Die Angabe der Enzymaktivität (U/ml) erfolgt in $\mu$Mol Substratumsatz pro min.

Beispiel 10: Präparation der in E. coli N 100/pRK 248/pJH 115 exprimierten Glucose-DH

Ein 10 l Laborfermenter wird mit 7,5 l LB-Medium beschickt, sterilisiert und im Anschluß mit 200 ml einer stationären Kultur des Überproduktionsstammes beimpft. Die Aufzucht der Zellen erfolgt bei 28° unter Belüftung. Nach dem Erreichen einer optischen Dichte von ca. 0,5 $A_{578}$-E wird die Temperatur auf 42° erhöht und die Kultur weitere 5 h unter Belüftung inkubiert.

Zur Präparation der Glucose-DH werden die Bakterien (ca. 10 g Feuchtgewicht) sedimentiert und im Anschluß durch Zermörsern mit der gleichen Menge Aluminiumoxid bei 4° aufgeschlossen. Nach Zugabe von 12,5 ml $P_{50}$-Puffer (50 mM Kaliumphosphat, pH 6,5; 0,1 mM 2-Mercaptoethanol; 2 $\mu$g Phenyl-Methyl-Sulfonylfluorid) und 50 $\mu$g DNase I wird 1 h bei 4° inkubiert. Die Entfernung des Aluminiumoxids erfolgt durch Zentrifugation bei 8 000 rpm und 4°. Der wässrige Überstand wird sodann durch Zentrifugation bei 15 000 rpm 30 min lang bei 4° geklärt.

Die Zellaufschlußlösung wird daraufhin mit 100 ml $P_{50}$-Puffer versetzt und auf eine DE-52-Säule aufgezogen. Nach Waschen mit 100 ml $P_{50}$ Puffer werden Proteine mit Hilfe eines linearen Gradienten von 0,05 auf 0,5 M KCl in $P_{50}$ Puffer eluiert und in Fraktionen von 5 ml gesammelt. Das Elutionsvolumen beträgt 1 l. Zur Erstellung eines Elutionsprofils wird die Absorption bei 280 nm sowie die Glucose-DH-Aktivität in den einzelnen Fraktionen bestimmt.

Die Ergebnisse der Proteinreinigung sind in folgender Tabelle zusammengefaßt.

## Reinigung der Glucose-DH

| Reinigungs-stufe | Gesamt-aktivität (U) | Gesamt-Protein (mg) | Spezifische Aktivität (U/mg) |
|---|---|---|---|
| Bakterien-aufschluß | 52 800 | 1 130 | 46,7 |
| Ammonium-sulfat-fällung | 49 500 | 1 000 | 49,5 |
| DE-52-Säule (Fraktion 58-68) | 6 880 | 29,4 | 234 |

Zur Überprüfung der Homogenität des Enzyms werden Proben auf einem 12,5 % SDS-Polyacrylamidgel analysiert.

**Patentansprüche**

**Patentansprüche für folgende Vertragsstaaten : DE, CH, LI, FR, GB, IT, NL, SE**

1. DNA-Molekül, kodierend für ein Polypeptid mit der biologischen Aktivität des Enzyms Glucose-Dehydrogenase, dadurch gekennzeichnet, daß es die Sequenz gemäß Fig. 1 enthält oder von dieser Sequenz abgeleitete Mutanten, kodierend für ein Polypeptid, weiches die gleiche Enzymaktivität wie die des Polypeptids gemäß Fig. 2 aufweist.

2. DNA-Molekül nach Anspruch 1, dadurch gekennzeichnet, daß es aus dem Genom eines Mikroorganismus, insbesondere Bacillus megaterium, stammt.

3. DNA-Molekül nach Anspruch 1 oder 2, dadurch gekennzeichnet, daß es eine Promoter-Sequenz von E. coli und / oder Bacillus megaterium enthält.

4. Plasmid mit der Bezeichnung pJH111 und der Hinterlegungsnummer DSM 4052P, enthaltend ein DNA-Molekül nach Anspruch 1 und ein Bacillus megaterium Promoter-System.

5. Plasmid mit der Bezeichnung pJH115 und der Hinterlegungsnummer DSM 4051P, enthaltend ein DNA-Molekül nach Anspruch 1 und ein E. coli Promoter-System.

**6.** Transformierter Wirtsorganismus, dadurch gekennzeichnet, daß er mindestens ein rekombinantes DNA-Molekül oder Plasmid nach einem der Ansprüche 3 bis 5 enthält.

**7.** Wirtsorganismus nach Anspruch 6, dadurch gekennzeichnet, daß er E. coli ist.

**8.** Wirtsorganismus nach Anspruch 7 mit der Bezeichnung E. coli N100/pRK248/pJH115 und der Hinterlegungsnummer DSM 4047, enthaltend das Plasmid gemäß Anspruch 5.

**9.** Polypeptid mit der biologischen Aktivität des Enzyms Glucose-Dehydrogenase und der Aminosäuresequenz gemäß Fig. 2.

**10.** Polypeptid mit der biologischen Aktivität des Enzyms Glucose-Dehydrogenase, dadurch gekennzeichnet, daß es von einem DNA-Molekül nach Anspruch 2 codiert worden ist.

**11.** Verfahren zur Herstellung von Glucose-Dehydrogenase durch Kultivierung eines mit rekombinanter DNA transformierten mikrobiellen Wirtsorganismus in einem Nährmedium und Isolierung der zur Expression gebrachten Polypeptide, dadurch gekennzeichnet, daß ein Wirtsorganismus gemäß einem der Ansprüche 6 bis 8 eingesetzt wird.

**12.** Verwendung eine Polypeptides nach Anspruch 9 oder 10 zur enzymatischen Glucosebestimmung.

**Patentansprüche für folgenden Vertragsstaat : ES**

**1.** Verfahren zur Herstellung von Glucose-Dehydrogenase durch Kultivierung eines mit rekombinanter DNA transformierten mikrobiellen Wirtsorganismus in einem Nährmedium und Isolierung der zur Expression gebrachten Polypeptide, dadurch gekennzeichnet, daß ein Wirtsorganismus eingesetzt wird, der mit einem rekombinanten DNA-Molekül oder Plasmid transformiert wurde, welches für ein Polypeptid mit der biologischen Aktivität des Enzyms Glucose-Dehydrogenase kodiert und die DNA-Sequenz gemäß Fig. 1 enthält oder von dieser Sequenz abgeleitete Mutanten, die für ein Polypeptid kodieren, das die gleiche Enzymaktivität wie die des Polypeptids gemäß Fig. 2 aufweist.

**2.** Verfahren nach Anspruch 1, dadurch gekennzeichnet, daß das DNA-Molekül mit dem der Wirtsorganismus transformiert wurde, aus dem Genom eines Mikroorganismus, insbesondere Bacillus megaterium, stammt.

**3.** Verfahren nach Anspruche 1 oder 2, dadurch gekennzeichnet, daß das DNA-Molekül eine Promoter-Sequenz von E. coli und/oder Bacillus megaterium enthält.

**4.** Verfahren nach einem der Ansprüche 1 bis 3, dadurch gekennzeichnet, daß ein Plasmid mit der Bezeichnung pJH115 und der Hinterlegungsnummer DSM 4051P eingesetzt wird.

**5.** Verfahren nach einem der Ansprüche 1 bis 4, dadurch gekennzeichnet, daß der Wirtsorganismus E. coli ist.

**6.** Verfahren nach Anspruch 5, dadurch gekennzeichnet, daß der Wirtsorganismus mit der Bezeichnung E. coli N100/pRK248/pJH115 und der Hinterlegungsnummer DSM 4047 eingesetzt wird.

**7.** Verwendung eines Polypeptides, das die biologische Aktivität des Enzyms Glucose-Dehydrogenase und die Aminosäuresequenz gemäß Fig. 2 aufweist oder das von einem DNA-Molekül kodiert wurde, welches die DNA-Sequenz gemäß Fig. 1 enthält oder von dieser Sequenz abgeleitete Mutanten, die für ein Polypeptid kodieren, das die gleiche Enzymaktivität wie die des Polypeptids gemäß Fig. 2 aufweist, zur enzymatischen Glucosebestimmung.

**Claims**

**Claims for the following Contracting States : CH, DE, FR, GB, IT, LI, NL, SE**

**1.** DNA molecule coding for a polypeptide having the biological activity of the enzyme glucose dehydrogenase, characterized in that it contains the sequence shown in Fig. 1, or mutants derived from this sequence and coding for a polypeptide which has the same enzyme activity as that of the polypeptide shown in Fig. 2.

2. DNA molecule according to Claim 1, characterized in that it originates from the genome of a microorganism, in particular Bacillus megaterium.

3. DNA molecule according to Claim 1 or 2, characterized in that it contains a promoter sequence from E. coli and/or Bacillus megaterium.

4. Plasmid having the name pJH111 and the depository number DSM 4052P, containing a DNA molecule according to Claim 1 and a Bacillus megaterium promoter system.

5. Plasmid having the name pJH115 and the depository number DSM 4051P, containing a DNA molecule according to Claim 1 and an E. coli promoter system.

6. Transformed host organism, characterized in that it contains at least one recombinant DNA molecule or plasmid according to one of Claims 3 to 5.

7. Host organism according to Claim 6, characterized in that it is E. coli.

8. Host organism according to Claim 7, having the name E. coli N100/pRK248/pJH115 and the depository number DSM 4047, containing the plasmid according to Claim 5.

9. Polypeptide having the biological activity of the enzyme glucose dehydrogenase and the amino acid sequence shown in Fig. 2.

10. Polypeptide having the biological activity of the enzyme glucose dehydrogenase, characterized in that it has been encoded by a DNA molecule according to Claim 2.

11. Process for the preparation of glucose dehydrogenase by cultivation, in a nutrient medium, of a microbial host organism which has been transformed with recombinant DNA, and isolation of the polypeptides whose expression has been brought about, characterized in that a host organism according to one of Claims 6 to 8 is used.

12. Use of a polypeptide according to Claim 9 or 10 for the enzymatic determination of glucose.

**Claims for the following Contracting State : ES**

1. Process for the preparation of glucose dehydrogenase by cultivation, in a nutrient medium, of a microbial host organism which has been transformed with recombinant DNA, and isolation of the polypeptides whose expression has been brought about, characterized in that a host organism is used which has been transformed with a DNA molecule or a plasmid coding for a polypeptide which has the biological activity of the enzyme glucose dehydrogenase and containing the DNA sequence shown in Fig. 1, or mutants derived from this sequence and coding for a polypeptide which has the same enzyme activity as that of the polypeptide shown in Fig. 2.

2. Process according to Claim 1, characterized in that the DNA molecule which has been used to transform the host organism originates from the genome of a microorganism, in particular Bacillus megaterium.

3. Process according to Claim 1 or 2, characterized in that the DNA molecule contains a promoter sequence from E.coli and/or Bacillus megaterium.

4. Process according to one of the Claims 1 to 3, characterized in that a plasmid having the name pJH 115 and the depository number DSM 4051P is used.

5. Process according to one of the Claims 1 to 4, characterized in that the host organism is E.coli.

6. Process according to Claim 5, characterized in that the host organism having the name E.coli N100/pRK248/pJH115 and the depository number DSM 4047 is used.

7. Use of a polypeptide which has the biological activity of the enzyme glucose dehydrogenase and the amino acid sequence shown in Fig.2, or has been coded by a DNA molecule which contains the DNA sequence shown in Fig. 1, or mutants derived from this sequence and coding for a polypeptide which has the same enzyme activity as that of the polypeptide shown in Fig. 2, for the enzymatic determination of glucose.

**Revendications**
**Revendications pour les Etats contractants suivants : CH, DE, FR, GB, IT, LI, NL, SE**

1. Molécule d'ADN codant pour un polypeptide ayant l'activité biologique de l'enzyme glucose déshydrogénase, caractérisée en ce qu'elle contient la séquence selon la Figure 1 où des mutants dérivés de cette séquence, codant pour un polypeptide qui présente la même activité enzymatique que celle du polypeptide selon la Figure 2.

2. Molécule d'ADN selon la revendication 1, caractérisée en ce qu'elle provient du génome d'un microorganisme, en particulier Bacillus megaterium.

3. Molécule d'ADN selon la revendication 1 ou 2, caractérisée en ce qu'elle contient une séquence promoteur d'E. Coli et/ou de Bacillus megaterium.

4. Plasmide désigné par pJH111 et déposé sous le numéro DSM 4052P, contenant une molécule d'ADN selon la revendication 1 et un système promoteur de Bacillus megaterium.

5. Plasmide désigné par pJH115 et déposé sous le numéro DSM 4051P, contenant une molécule d'ADN selon la revendication 1 et un système promoteur d'E. Coli.

6. Organisme hôte transformé, caractérisé en ce qu'il contient au moins une molécule d'ADN recombinant ou un plasmide selon une des revendications 3 à 5.

7. Organisme hôte selon la revendication 6, caractérisé en ce qu'il s'agit de E. Coli.

8. Organisme hôte selon la revendication 7, appelé E. Coli N100/pRK248/pJH115 et déposé sous le numéro DSM 4047, contenant le plasmide selon la revendication 5.

9. Polypeptide ayant l'activité biologique de l'enzyme-glucose déshydrogénase et la séquence d'acides aminés selon la Figure 2.

10. Polypeptide ayant l'activité biologique de l'enzyme glucose-déshydrogénase, caractérisé en ce qu'il est codé par une molécule d'ADN selon la revendication 2.

11. Procédé de préparation de glucose-déshydrogénase par culture d'un organisme hôte microbien transformé avec de l'ADN recombinant dans un milieu nutritif et isolement des polypeptides amenés à l'expression, caractérisé en ce qu'on utilise un organisme hôte selon une des revendications 6 à 8.

12. Application d'un polypeptide selon la revendication 9 ou 10 à la détermination enzymatique glucose.

**Revendications pour l'Etat contractant suivant : ES**

1. Procédé de préparation de glucose-déshydrogénase par culture d'un microorganisme hôte microbien transformé avec un ADN recombinant dans un milieu nutritif et isolement du polypeptide amené à l'expression, caractérisé en ce qu'on utilise un organisme hôte qui a été transformé avec une molécule d'ADN recombinant ou un plasmide, qui code pour un polypeptide ayant l'activité biologique de l'enzyme glucose-déshydrogénase et qui contient la séquence d'ADN selon la Figure 1 où des mutants dérivés de cette séquence, qui codent pour un polypeptide qui présente la même activité enzymatique que celle du polypeptide selon la Figure 2.

**2.** Procédé selon la revendication 1, caractérisé en ce que la molécule d'ADN avec laquelle l'organisme hôte a été transformé provient du génome d'un microorganisme, en particulier Bacillus megaterium.

**3.** Procédé selon les revendications 1 ou 2, caractérisé en ce que la molécule d'ADN contient une séquence promoteur de E. Coli et/ou de Bacillus megaterium.

**4.** Procédé selon l'une des revendications 1 a 3, caractérisé en ce que l'on utilise un plasmide appelé pJH115 et déposé sous le numéro DSM 4051P.

**5.** Procédé selon l'une des revendications 1 a 4, caractérisé en ce que l'organisme hôte est E. Coli.

**6.** Procédé selon la revendication 5, caractérisé en ce qu'on utilise l'organisme hôte appelé E. Coli N100/pRK248/pJH115 et déposé sous le numéro DSM 4047.

**7.** Application d'un polypeptide qui présente l'activité biologique de l'enzyme glucose-déshydrogénase et la séquence d'acides aminés selon la Figure 2 où est codé par une molécule d'ADN qui contient la séquence d'ADN selon la Figure 1 où des mutants dérivés de cette séquence, qui codent pour un polypeptide présentant la même activité enzymatique que celle du polypeptide selon la Figure 2, pour la détermination enzymatique du glucose.

18

# FIG. 1

DNA-Sequenz des Glucosedehydrogenase-Gens
aus Bacillus megaterium

```
ATG-TAT-ACA-GAT-TTA-AAA-GAT-AAA-GTA-GTT-GTA-ATT-
ACA-GGT-GGA-TCA-ACA-GGT-TTA-GGA-CGC-GGA-ATG-GCT-
GTT-CGT-TTC-GGT-CAA-GAA-GAA-GCA-AAA-GTT-GTT-ATT-
AAC-TAT-TAC-AAC-AAT-GAA-GAA-GAA-GCT-CTA-GAT-GCG-
AAA-AAA-GAA-GTA-GAA-GAA-GCA-GGC-GGA-CAA-GCA-ATC-
ATC-GTT-CAA-GGC-GAT-GTA-ACA-AAA-GAA-GAA-GAC-GTT-
GTA-AAT-CTT-GTT-CAA-ACA-GCT-ATT-AAA-GAA-TTT-GGT-
ACA-TTA-GAC-GTA-ATG-ATT-AAC-AAC-GCT-GGT-GTT-GAA-
AAC-CCA-GTT-CCT-TCT-CAT-GAG-CTA-TCT-CTA-GAT-AAC-
TGG-AAC-AAA-GTT-ATT-GAT-ACA-AAC-TTA-ACA-GGT-GCA-
TTC-TTA-GGA-AGC-CGT-GAA-GCA-ATT-AAA-TAC-TTC-GTT-
GAA-AAC-GAC-ATT-AAA-GGA-AAT-GTT-ATC-AAC-ATG-TCT-
AGC-GTT-CAC-GAA-ATG-ATT-CCT-TGG-CCA-TTA-TTT-GTT-
CAC-TAC-GCA-GCA-AGT-AAA-GGC-GGT-ATG-AAA-CTA-ATG-
ACG-GAA-ACA-TTG-GCT-CTT-GAA-TAT-GCG-CCA-AAA-GGT-
ATT-CGC-GTA-AAT-AAT-ATT-GGA-CCA-GGT-GCG-ATG-AAC-
ACA-CCA-ATT-AAC-GCA-GAG-AAA-TTT-GCA-GAT-CCA-GAA-
CAA-CGT-GCA-GAC-GTA-GAA-AGC-ATG-ATT-CCA-ATG-GGT-
TAC-ATC-GGT-AAA-CCA-GAA-GAA-GTA-GCA-GCA-GTT-GCA-
GCA-TTC-TTA-GCT-TCA-TCA-CAA-GCA-AGC-TAT-GTA-ACA-
GGT-ATT-ACA-TTA-TTT-GCA-GAT-GGC-GGT-ATG-ACG-AAA-
TAC-CCT-TCT-TTC-CAA-GCA-GGA-AGA-GGC-TAA-TAG
```

# FIG.2

Aminosäure - Sequenz des Expressionsproduktes
von pJH111 bzw. pJH115.


Met-Tyr-Thr-Asp-Leu-Lys-Asp-Lys-Val-Val-Val-Ile-
Thr-Gly-Gly-Ser-Thr-Gly-Leu-Gly-Arg-Ala-Met-Ala-
Val-Arg-Phe-Gly-Gln-Glu-Glu-Ala-Lys-Val-Val-Ile-
Asn-Tyr-Tyr-Asn-Asn-Glu-Glu-Glu-Ala-Leu-Asp-Ala-
Lys-Lys-Glu-Val-Glu-Glu-Ala-Gly-Gly-Gln-Ala-Ile-
Ile-Val-Gln-Gly-Asp-Val-Thr-Lys-Glu-Glu-Asp-Val-
Val-Asn-Leu-Val-Gln-Thr-Ala-Ile-Lys-Glu-Phe-Gly-
Thr-Leu-Asp-Val-Met-Ile-Asn-Asn-Ala-Gly-Val-Glu-
Asn-Pro-Val-Pro-Ser-His-Glu-Leu-Ser-Leu-Asp-Asn-
Trp-Asn-Lys-Val-Ile-Asp-Thr-Asn-Leu-Thr-Gly-Ala-
Phe-Leu-Gly-Ser-Arg-Glu-Ala-Ile-Lys-Tyr-Phe-Val-
Glu-Asn-Asp-Ile-Lys-Gly-Asn-Val-Ile-Asn-Met-Ser-
Ser-Val-His-Glu-Met-Ile-Pro-Trp-Pro-Leu-Phe-Val-
His-Tyr-Ala-Ala-Ser-Lys-Gly-Gly-Met-Lys-Leu-Met-
Thr-Glu-Thr-Leu-Ala-Leu-Gly-Tyr-Ala-Pro-Lys-Gly-
Ile-Arg-Val-Asn-Asn-Ile-Gly-Pro-Gly-Ala-Met-Asn-
Thr-Pro-Ile-Asn-Ala-Glu-Lys-Phe-Ala-Asp-Pro-Glu-
Gln-Arg-Ala-Asp-Val-Glu-Ser-Met-Ile-Pro-Met-Gly-
Tyr-Ile-Gly-Lys-Pro-Glu-Glu-Val-Ala-Ala-Val-Ala-
Ala-Phe-Leu-Ala-Ser-Ser-Gln-Ala-Ser-Tyr-Val-Thr-
Gly-Ile-Thr-Leu-Phe-Ala-Asp-Gly-Gly-Met-Thr-Lys-
Tyr-Pro-Ser-Phe-Gln-Ala-Gly-Arg-Gly

FIG.3    Restriktionskarte des Plasmid-Vektors
pJH107.

ClaI  Eco RI        ClaI        SphI

SalI        Eco RI  HindⅢ  SphI      HindⅢ    SalI

← − 5800 bp →

FIG.4    Restriktionskarte des Plasmid-Vektors
pJH108.

ClaI  SphI  HindⅢ

Eco RI  HindⅢ      ClaI    HindⅢ        SphI        Eco RI

← 3000 bp →

# FIG.5

Sequenzierungsstrategie des 1126 bp-Fragments aus pJH111.

# FIG.6

Konstruktion des Plasmid-Vektors pJH115.

FIG.7     Proteinsequenzvergleich zwischen Glucose-DH,
          basierend auf 1100 bp-Fragment (a), und
          Glucose-DH, basierend auf 2100 bp-Fragment
          (b; bekannte Sequenz).


a     Met-Tyr-Thr-Asp-Leu-Lys-Asp-Lys-Val-Val-Val-Ile-
      Thr-Gly-Gly-

b     Met-Tyr-Lys-Asp-Leu-Glu-Gly-Lys-Val-Val-Val-Ile-
      Thr-Gly-Ser-


a     Ser-Thr-Gly-Leu-Gly-Arg-Ala-Met-Ala-Val-Arg-Phe-
      Gly-Gln-Glu-

b     Ser-Thr-Gly-Leu-Gly-Lys-Ser-Met-Ala-Ile-Arg-Phe-
      Ala-Thr-Glu-


a     Glu-Ala-Lys-Val-Val-Ile-Asn-Tyr-Tyr-Asn-Asn-Glu-
      Glu-Glu-Ala-

b     Lys-ala-Lys-Val-Val-Val-Asn-Tyr-Arg-Ser-Lys-Glu-
      Asp-Glu-Ala-

**FIG. 8**   Wachstums- und Expressionskurve
von E. coli   N100/pRK248/pJH115.

**FIG.9**  Überproduktion von Glucosedehydrogenase in E. coli N100/pRK248/pJH115.

**FIG.10**  Autoradiogramm der Hybridisierung chromosomaler DNA aus Bacillus megaterium mit dem radioaktiv markierten 1100 bp-DNA-Fragment aus pJH111 bzw. pJH115 nach 12h Exposition.

# FIG.11  Restriktionskarte von pJH211.

HindIII      EcoRI    AccI    HindIII     AccI / BamHI / EcoRI

← 2 100 bp →

lac z

O/P

Ap$^r$

p J H 211
4.8 kb

ori

# FIG.12  Restriktionskarte von pJH215.

EcoRI   BamHI   HindIII   StuI

O$_L$P$_L$

gdh2

bla

p J H 215
6.9 kb

HindIII

galk

ori

BamHI

BamHI